# EUROPEAN PATENT APPLICATION

(11) **EP 4 082 540 A1**
(43) Date of publication of application: **02.11.2022**
(21) Application number: 20907334.5
(22) Date of filing: 25.12.2020
(51) Int. Cl.: A61K 31/19, A23L 2/52, A23L 33/105, A61P 1/16, A61P 3/06, A61P 3/10

(54) **NOVEL USE OF STEVIOL**

(30) Priority: 27.12.2019 JP 2019238968
(71) Applicant: Suntory Holdings Limited, Osaka 530-8203 (JP)
(72) Inventor: YOSHIDA Junki, Kawasaki-shi, Kanagawa 211-0067 (JP); OHKURI Tadahiro, Kawasaki-shi, Kanagawa 211-0067 (JP); NAGAO Koji, Kawasaki-shi, Kanagawa 211-0067 (JP); YOKOO Yoshiaki, Kawasaki-shi, Kanagawa 211-0067 (JP)
(74) Representative: Mewburn Ellis LLP
(86) International application number: PCT/JP2020/049039
(87) International publication number: WO 2021/132697

(57) **Abstract**

The present invention provides a composition which contains a steviol and/or steviol glycoside, and is for inhibiting the accumulation of fat, promoting lipolysis, reducing blood glucose level, enhancing the intake of glucose, improving the metabolism of glucose, or treating alcoholic liver disease or non-alcoholic fatty liver disease.

## Description

### TECHNICAL FIELD

The present invention relates to novel use of steviol, particularly, use of steviol relating to promotion of lipolysis and suppression of fat accumulation, reduction in blood glucose level, promotion of sugar uptake and/or improvement in sugar metabolism, and treatment of alcoholic liver disease or nonalcoholic fatty liver disease, etc.

### BACKGROUND ART

In modern society with well-developed transportation infrastructure where high-calorie highly palatable foods can be easily obtained, and further, the proportion of sedentary workers has increased, energy intake often tends to exceed expenditure, which in all aspects facilitates fat accumulation in the body. Excessive accumulation of fat causes obesity, fatty liver, and the like. Besides, the relation of obesity to the development of type 2 diabetes mellitus, coronary heart disease, respiratory sleep disorder such as sleep apnea syndrome, and osteoarthritis has also been pointed out (Non-Patent Literature 1). Although excessive accumulation of fat may be coped with by lifestyle modification such as devised dietary constituents or encouragement of exercise, not everyone can help themselves in such a way. Pathological accumulation of fat may be medically treated. For example, in the United States which has many obese patients, appetite suppressants such as phentermine or diethylpropion, orlistat which suppresses fat absorption from the intestine, and the like are employed in the treatment of obesity (Non-Patent Literature 2).

### CITATION LIST

### PATENT LITERATURE

Non-Patent Literature 1: Kopelman, Nature. 2000;404(6778):635-43
Non-Patent Literature 2: Gadde et al., Clin Chem. 201864(1):118-129

### SUMMARY OF INVENTION

### TECHNICAL PROBLEM

The development of a further approach of improving excessive accumulation of fat is expected.

### MEANS FOR SOLVING THE PROBLEMS

In one embodiment, the present invention provides the following.
[1] A composition for use in the suppression of fat accumulation and/or in the promotion of lipolysis, the composition comprising steviol and/or steviol glycoside.
[1-1] A composition for use in the suppression of fat accumulation and/or in the promotion of lipolysis, the composition comprising steviol and/or steviol glycoside as active ingredient.
[2] The composition for use according to [1] or [1-1] for use in the prevention of obesity, in the improvement of obesity and/or in the reduction of body fat.
[3] The composition for use according to [1], [1-1] or [2] for a subject free of diabetes mellitus that responds to insulin therapy.
[4] A composition for use in the reduction of blood glucose level, in the promotion of sugar uptake and/or in the improvement of sugar metabolism in a subject having insulin resistance, the composition comprising steviol and/or steviol glycoside.
[4-1] A composition for use in the reduction of blood glucose level, in the promotion of sugar uptake and/or in the improvement of sugar metabolism in a subject having insulin resistance, the composition comprising steviol and/or steviol glycoside as active ingredient.
[5] A composition for use in the treatment of alcoholic liver disease or nonalcoholic fatty liver disease, the composition comprising steviol and/or steviol glycoside.
[5-1] A composition for use in the treatment of alcoholic liver disease or nonalcoholic fatty liver disease, the composition comprising steviol and/or steviol glycoside as active ingredient.
[6] The composition for use according to any one of [1] to [5] for oral ingestion.
[7] The composition for use according to any one of [1] to [6] which is a beverage.

### ADVANTAGEOUS EFFECTS OF INVENTION

The present invention provides a further approach for promoting lipolysis, suppressing fat accumulation and improving sugar uptake, and options for preserving or enhancing health and preventing or improving a condition involving fat accumulation are increased. Furthermore, steviol is also useful in reduction in already accumulated fat because of its lipolysis-promoting effect. Moreover, steviol promotes sugar uptake in cells and as such, can also be employed in use such as reduction in blood glucose level or improvement in sugar metabolism. Also, steviol can be orally administered as steviol glycoside which may be employed as a sweetener and has high compatibility with foods. Therefore, steviol can be ingested more conveniently as compared with conventional drugs, and can contribute to health enhancement for a wider range of people.

### BRIEF DESCRIPTION OF DRAWINGS

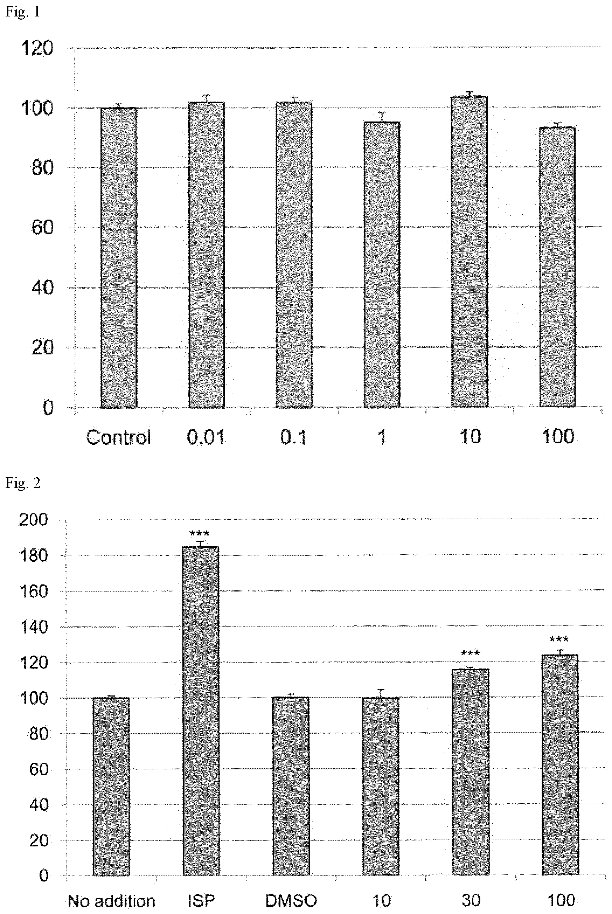

Figure 1 is a diagram showing results of a cytotoxicity test of steviol to adipocytes (n = 3). The ordinate depicts absorbance difference (relative viable cell count (Control = 100%)), and the numerical values on the abscissa depict the concentrations (µg/mL) of steviol.
Figure 2 is a diagram showing results of a lipolysis test of steviol in adipocytes (n = 5). The numerical values on the abscissa depict the concentrations (µg/mL) of steviol, and ISP depicts positive control group. The ordinate depicts a relative lipolysis level when the level of negative control (No addition vs ISP, DMSO vs steviol) is defined as 100%. ^{∗∗∗} represents P < 0.001 based on the Student's t test.

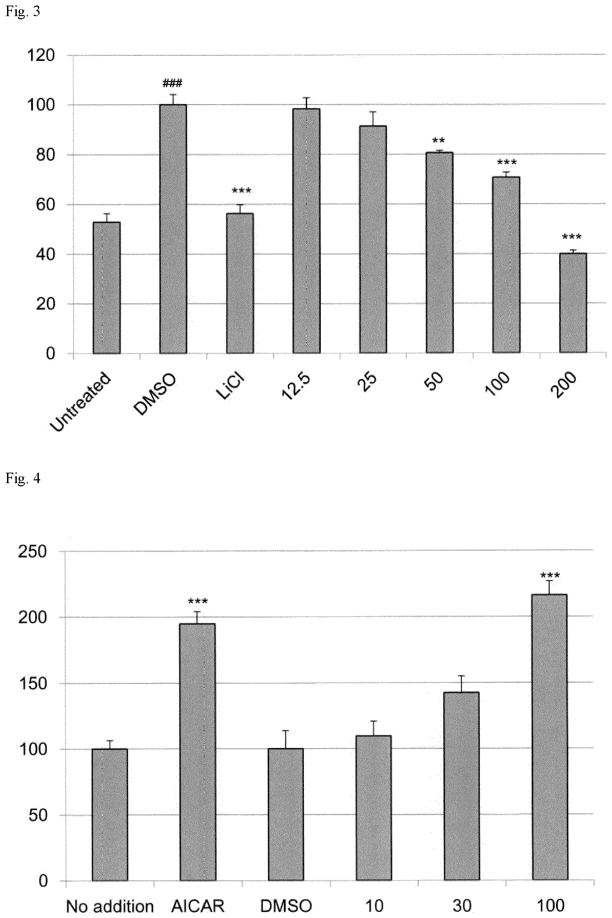

Figure 3 is a diagram showing results of a fat accumulation test of steviol in adipocytes (n = 5). The numerical values on the abscissa depict the concentrations (µg/mL) of steviol, and LiCl depicts positive control group. The ordinate depicts a relative fat accumulation level when the level of negative control (DMSO) is defined as 100%. ### represents P < 0.001 based on the Student's t test. ^{∗∗∗} and ^{∗∗} represent P < 0.001 and P < 0.005, respectively, based on the Dunnett test.
Figure 4 is a diagram showing results of a sugar uptake test of steviol in adipocytes (n = 5). The numerical values on the abscissa depict the concentrations (µg/mL) of steviol, and AICAR depicts positive control group. The ordinate depicts relative luminescence intensity (relative intracellular 2DG6P level) when the level of negative control (No addition vs AICAR, DMSO vs steviol) is defined as 100%. ^{∗∗∗} represents P < 0.001 based on the Student's t test.

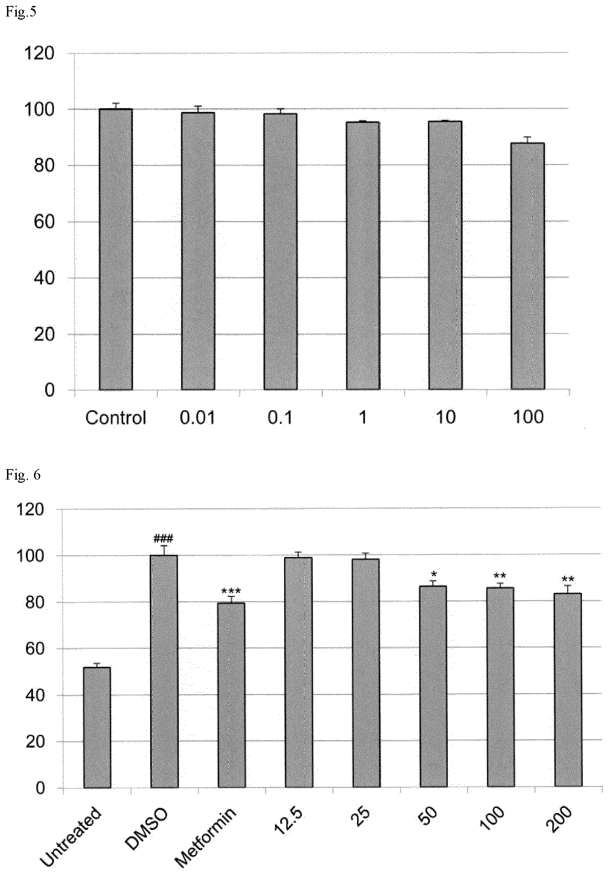

Figure 5 is a diagram showing results of a cytotoxicity test of steviol to hepatocytes (n = 3). The ordinate depicts absorbance difference (relative viable cell count (Control = 100%)), and the numerical values on the abscissa depict the concentrations (µg/mL) of steviol.
Figure 6 is a diagram showing results of a fat accumulation test of steviol in hepatocytes (n = 5). The numerical values on the abscissa depict the concentrations (µg/mL) of steviol, and Metformin depicts positive control group. The ordinate depicts a relative fat accumulation level when the level of negative control (DMSO) is defined as 100%. ### represents P < 0.001 based on the Student's t test. ^{∗∗∗}, ^{∗∗} and ^{∗} represent P < 0.001, P < 0.01 and P < 0.05, respectively, based on the Dunnett test.

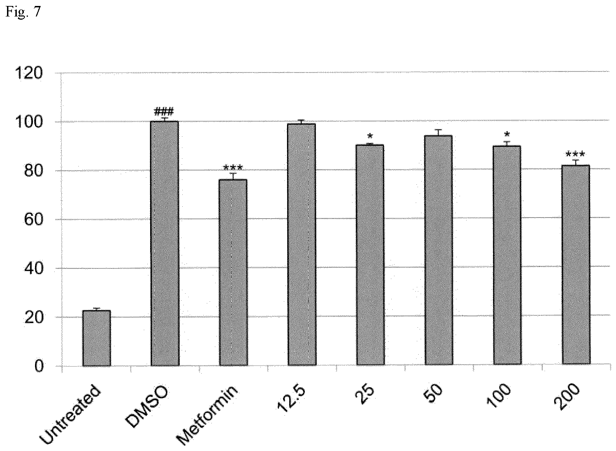

Figure 7 is a diagram showing results of a TG accumulation test of steviol in hepatocytes (n = 5). The numerical values on the abscissa depict the concentrations (µg/mL) of steviol, and Metformin depicts positive control group. The ordinate depicts a relative TG accumulation level when the level of negative control (DMSO) is defined as 100%. ### represents P < 0.001 based on the Student's t test. ^{∗∗∗} and ^{∗} represent P < 0.001 and P < 0.05, respectively, based on the Dunnett test.

### DESCRIPTION OF EMBODIMENTS

The present invention will now be described in detail. The following embodiments are provided for illustrating the present invention and are not intended to limit the present invention only thereto. The present invention may be implemented in various forms, without departing from the spirit of the present invention.

Note that all documents, as well as laid-open application publications, patent application publications, and other patent documents cited herein shall be incorporated herein by reference. The present specification incorporates the contents of the specification and the drawings of Japanese Patent Application No. 2019-238968, filed on December 27, 2019, from which the present application claims priority.

### <Composition for promotion of lipolysis/suppression of fat accumulation>

One embodiment of the present invention relates to a composition for use in the suppression of fat accumulation and/or in the promotion of lipolysis (hereinafter, also referred to as the "composition for use in the promotion of lipolysis/suppression of fat accumulation of the present invention" or the "composition A of the present invention"), the composition comprising steviol and/or a source of steviol (e.g., steviol glycoside).

The present inventors have found for the first time that steviol has a fat accumulation-suppressing effect and a lipolysis-promoting effect, and completed the composition A of the present invention. Thus, steviol and a source thereof can act as an active ingredient in the composition A of the present invention. The action as an active ingredient in the composition A of the present invention can be confirmed by, for example, more suppressed fat accumulation and/or more promoted lipolysis by the use of the composition A of the present invention than the use of a control composition when the composition A of the present invention is compared with the control composition which has the same composition as that of the composition A of the present invention except that the control composition is free of steviol and/or a source thereof.

Steviol is an aglycon of steviol glycoside contained in Stevia rebaudiana.

The source of steviol includes a substance capable of providing steviol by the action of an in vivo enzyme (which includes an enzyme produced by an in vivo bacterium or the like) when administered to a living body. Examples of the source of steviol include, but are not limited to, steviol glycoside and glucuronic acid conjugate of steviol (steviol glucuronide). The steviol glycoside is glycoside in which steviol is bonded to one or more sugars (e.g., glucose, rhamnose, and xylose). Non-limiting examples of steviol glycoside include, for example, rebaudioside A, rebaudioside B, rebaudioside D, rebaudioside E, rebaudioside F, rebaudioside I, rebaudioside J, rebaudioside K, rebaudioside M, rebaudioside N, rebaudioside O, rebaudioside Q, rebaudioside R, dulcoside A, rubusoside, steviolmonoside, steviolbioside and stevioside. In a preferred embodiment, the steviol glycoside includes a substance having good quality of taste, for example, rebaudioside D and rebaudioside M. In the present specification, rebaudioside is also abbreviated to "Reb", "Reb." or "R". In the present specification, for example, rebaudioside A is also referred to as "RebA", "Reb.A" or "RA". The steviol glycoside, when orally ingested, is degraded into steviol by large intestinal bacteria and absorbed into the body. Thus, the steviol glycoside serves as a source of steviol via the large intestine.

Commercially available steviol and source of steviol (e.g., steviol glycoside) may be employed. Steviol and a source of steviol extracted, purified, etc. from Stevia rebaudiana may be employed. An approach of extracting and purifying steviol and steviol glycoside from Stevia rebaudiana is known. Steviol and steviol glycoside can be extracted from a fresh leaf or a dried leaf of Stevia rebaudiana with a suitable solvent (an aqueous solvent such as water or an organic solvent such as alcohol, ether and acetone) (for extraction conditions, etc., see a method described in, for example, Ohta et al., J. Appl. Glycosci. 2010;57(3):199-209 or WO 2010/038911). Steviol and each type of steviol glycoside can be purified from the extracts thus obtained by use of a method known in the art such as gradient of ethyl acetate or any other organic solvent:water, high-performance liquid chromatography (HPLC), gas chromatography, time-of-flight mass spectrometry (TOF-MS), or ultra-performance liquid chromatography (UPLC). Steviol can be obtained by, for example, deglycosylation of steviol glycoside (e.g., Shibata et al., Plant Physiol. 1991;95(1):152-6). For steviol glycoside, particularly, with a small content in Stevia rebaudiana, it is possible to elevate its concentration by bioconversion which allows glycosyltransferase (e.g., UDP-sugar dependent glycosyltransferase (UGT)) to act on Stevia rebaudiana extracts containing steviol glycoside.

In one embodiment, the steviol glycoside to be employed in the composition A of the present invention is extracted and purified from Stevia rebaudiana without bioconversion. In another embodiment, the steviol glycoside to be employed in the composition A of the present invention is extracted and purified from Stevia rebaudiana by an approach including bioconversion.

It is more preferred that steviol and the source of steviol (e.g., steviol glycoside) to be employed in the composition A of the present invention should have a higher purity. Examples of the purity of steviol or the source of steviol (e.g., steviol glycoside) to be employed in the present invention include 90% or higher, 90.5% or higher, 91% or higher, 91.5% or higher, 92% or higher, 92.5% or higher, 93% or higher, 93.5% or higher, 94% or higher, 94.5% or higher, 95% or higher, 95.5% or higher, 96% or higher, 96.5% or higher, 97% or higher, 97.5% or higher, 98% or higher, 98.5% or higher, 99% or higher, and 99.5% or higher. For example, a peak area ratio in chromatography such as HPLC or GC may be used as an index for the purity of steviol or the source of steviol (e.g., steviol glycoside).

The composition A of the present invention may comprise steviol, a source of steviol (e.g., steviol glycoside), or a combination of steviol and a source of steviol (e.g., steviol glycoside). The blending ratio of steviol, the source of steviol (e.g., steviol glycoside), or the combination of steviol and the source of steviol (e.g., steviol glycoside) in the composition A of the present invention can be, for example, 1 to 500,000, 1 to 400,000, 1 to 300,000, 1 to 200,000, 1 to 100,000, 1 to 80,000, 1 to 60,000, 1 to 40,000, 1 to 20,000, 1 to 10,000, 1 to 8,000, 1 to 6,000, 1 to 4,000, 1 to 2,000, 1 to 1500, 1 to 1200, 5 to 1200, 1 to 1000, 5 to 1000, 10 to 1000, 1 to 900, 5 to 900, 10 to 900, 15 to 900, 20 to 900, 25 to 900, 30 to 900, 35 to 900, 40 to 900, 45 to 900, 50 to 900, 55 to 900, 1 to 800, 5 to 800, 10 to 800, 15 to 800, 20 to 800, 25 to 800, 30 to 800, 35 to 800, 40 to 800, 45 to 800, 50 to 800, 55 to 800, 1 to 700, 5 to 700, 10 to 700, 15 to 700, 20 to 700, 25 to 700, 30 to 700, 35 to 700, 40 to 700, 45 to 700, 50 to 700, 55 to 700, 1 to 600, 5 to 600, 10 to 600, 15 to 600, 20 to 600, 25 to 600, 30 to 600, 35 to 600, 40 to 600, 45 to 600, 50 to 600, 55 to 600, 1 to 500, 5 to 500, 10 to 500, 15 to 500, 20 to 500, 25 to 500, 30 to 500, 35 to 500, 40 to 500, 45 to 500, 50 to 500, 55 to 500, 1 to 400, 5 to 400, 10 to 400, 15 to 400, 20 to 400, 25 to 400, 30 to 400, 35 to 400, 40 to 400, 45 to 400, 50 to 400, 55 to 400, 1 to 300, 5 to 300, 10 to 300, 15 to 300, 20 to 300, 25 to 300, 30 to 300, 35 to 300, 40 to 300, 45 to 300, 50 to 300, 55 to 300, 1 to 250, 5 to 250, 10 to 250, 15 to 250, 20 to 250, 25 to 250, 30 to 250, 35 to 250, 40 to 250, 45 to 250, 50 to 250, 55 to 250, 1 to 200, 5 to 200, 10 to 200, 15 to 200, 20 to 200, 25 to 200, 30 to 200, 35 to 200, 40 to 200, 45 to 200, 50 to 200, 55 to 200, 1 to 180, 5 to 180, 10 to 180, 15 to 180, 20 to 180, 25 to 180, 30 to 180, 35 to 180, 40 to 180, 45 to 180, 50 to 180, 55 to 180, 1 to 550, 1 to 540, 1 to 530, 1 to 520, 1 to 510, 1 to 505, 1 to 495, 1 to 490, 5 to 550, 5 to 540, 5 to 530, 5 to 520, 5 to 510, 5 to 505, 5 to 500, 5 to 495, 5 to 490, 10 to 550, 10 to 540, 10 to 530, 10 to 520, 10 to 510, 10 to 505, 10 to 495, 10 to 490, 15 to 550, 15 to 540, 15 to 530, 15 to 520, 15 to 510, 15 to 505, 15 to 495 or 15 to 490 ppm based on the total mass of the composition A.

Lipolysis means decomposition of accumulated fat in cells having fat accumulation ability (e.g., adipocytes, hepatocytes, and muscle cells), followed by release to the outside of the cells (also called fat mobilization). The promotion of lipolysis by the composition A of the present invention can be evaluated by, for example, a larger amount of lipolysis product released into a medium by the action of the composition A of the present invention on cells having fat accumulation ability cultured under fat accumulation conditions, than that without the action of the composition A of the present invention. In a particular embodiment, the promotion of lipolysis can be evaluated, as described in Examples mentioned later, by culturing cells having fat accumulation ability under fat accumulation conditions (e.g., in a medium for preadipocyte differentiation or in a medium supplemented with a fatty acid such as oleic acid), then incubating the cells with the composition A of the present invention, and after a predetermined time (e.g., 24 hours later), detecting and quantifying a lipolysis product (fatty acid, glycerol, etc.) released into the medium with a detection reagent or the like. The degree of promotion of lipolysis may be, for example, 102% or more, 105% or more, 108% or more, 110% or more, 112% or more, 115% or more, 118% or more, 120% or more, 122% or more, 125% or more, 128% or more, or 130% or more in terms of the amount of a lipolysis product in cells treated with the composition A of the present invention when the amount of a lipolysis product released under the same conditions as above except that the composition A of the present invention is not employed is defined as 100%. A higher value is more preferred.

Fat accumulation means uptake of an extracellular free fatty acid or the like by cells having fat accumulation ability, followed by lipogenesis and accumulation in the cells. The suppression of fat accumulation by the composition A of the present invention can be evaluated by, for example, a smaller intracellular fat level (particularly, triglyceride level) as compared with the case where the composition A of the present invention is not employed in cells under fat accumulation conditions. In a particular embodiment, the suppression of fat accumulation can be evaluated, as described in Examples mentioned later, by culturing cells having fat accumulation ability under fat accumulation conditions together with the composition A of the present invention, and after a predetermined time (e.g., 10 days later), detecting fat accumulated in the cells by oil red staining or with any other detection reagent or the like, followed by quantification by image analysis or the like. The degree of suppression of fat accumulation may be, for example, 95% or less, 90% or less, 85% or less, 80% or less, 75% or less, 70% or less, 65% or less, 60% or less, 55% or less, 50% or less, 45% or less, or40% or less in terms of a fat level in cells treated with the composition A of the present invention when a fat level obtained under the same conditions as above except that the composition A of the present invention is not employed is defined as 100%. A lower value is more preferred.

The composition A of the present invention can be employed in treatment of various conditions involving fat accumulation. Thus, the present invention also relates to a composition for treating a condition involving fat accumulation, the composition comprising steviol and/or a source of steviol (e.g., steviol glycoside). In this context, the condition includes a disease, and a condition that is not far enough to be diagnosed as a disease, but is not healthy. Examples of the condition involving fat accumulation include, but are not limited to, obesity, fatty liver, type 2 diabetes mellitus, coronary heart disease, arteriosclerosis, arrhythmia, cardiomyovasculopathy (triglyceride deposit cardiomyovasculopathy (TGCV), etc.), cardiac sudden death, respiratory sleep disorder (sleep apnea syndrome, etc.), osteoarthritis, hypertension, insulin resistance, leptin resistance, hyperlipidemia, eccentric cardiac hypertrophy, concentric cardiac hypertrophy, heart failure, and metabolic syndrome (Kopelman, Nature. 2000;404(6778):635-43, Izquierdo et al., Nutrients. 2019;11(11). pii: E2704, Hirano, J Atheroscler Thromb. 2009;16(5):702-5, Csige et al., J Diabetes Res. 2018 Nov 4;2018). The fatty liver includes alcoholic fatty liver (which is an alcoholic liver disease) and nonalcoholic fatty liver disease (NAFLD). NAFLD includes simple fatty liver and nonalcoholic steatohepatitis (NASH). In one embodiment, the condition involving fat accumulation is a disease other than diabetes mellitus or its complications. In another embodiment, the condition involving fat accumulation is a disease on which insulin therapy has low efficacy. In another embodiment, the composition A of the present invention is employed in a subject without diabetes mellitus that responds to insulin therapy (e.g., type 1 diabetes mellitus). In another embodiment, the composition A of the present invention is employed in a subject that manifests insulin resistance. In another embodiment, the composition A of the present invention is employed for a method for treating a condition involving fat accumulation without control of energy intake. The phrase "without control of energy intake" includes, for example, the absence of diet restriction and continuation of usual eating habits (e.g., eating habits before the start of treatment). The lipolysis-promoting effect and the fat accumulation-suppressing effect of steviol revealed this time are not mediated by insulin. Therefore, the composition A of the present invention can exert its effects in an insulin-independent manner. Thus, the composition A of the present invention may be employed even for the disease, as described above, on which insulin cannot be expected to have a therapeutic effect. Furthermore, unlike many anti-obesity drugs, the composition A of the present invention acts directly on cells having fat accumulation ability, instead of suppressing appetite, and can therefore treat a condition involving fat accumulation without impairing enjoyment of eating and drinking.

The composition A of the present invention can also be employed for reducing body fat with the aim of health enhancement, weight loss, prevention of obesity, maintenance or improvement of a body shape, etc. This use includes not only medical use but nonmedical use such as cosmetic use, sport use, and fitness use.

### <Composition for promotion of sugar uptake>

Another embodiment of the present invention relates to a composition for use in the promotion of sugar uptake, in the reduction of blood glucose level and/or in the improvement of sugar metabolism (hereinafter, also referred to as the "composition for use in the promotion of sugar uptake of the present invention" or the "composition B of the present invention"), the composition comprising steviol and/or a source of steviol (e.g., steviol glycoside).

The present inventors have found for the first time that steviol has a sugar uptake-promoting effect, and completed the composition B of the present invention. Thus, steviol and a source thereof can act as an active ingredient in the composition B of the present invention. The action as an active ingredient in the composition B of the present invention can be confirmed by, for example, more promoted sugar uptake by the use of the composition B of the present invention than the use of a control composition when the composition B of the present invention is compared with the control composition which has the same composition as that of the composition B of the present invention except that the control composition is free of steviol and/or a source thereof.

The origin, purity, the amount blended, etc., of steviol and the source of steviol (e.g., steviol glycoside) in the composition B of the present invention are as described above about the composition A of the present invention.

Sugar uptake means cellular uptake of sugar (glucose) by cells having sugar uptake ability (e.g., adipocytes). The promotion of sugar uptake by the composition B of the present invention can be evaluated by change in index for sugar uptake as compared with the case where the composition B of the present invention is not employed. Examples of the index for sugar uptake include intracellular amount of a glucose-related substance (e.g., 2-deoxyglucose (2DG) and 2-deoxy-2-[(7-nitro-2,1,3-benzoxadiazol-4-yl)amino]-D-glucose (2-NBDG)) which does not undergo metabolism by the glycolysis system, or a derivative thereof (e.g., 2-deoxyglucose-6-phosphate (2DG6P)) after administration of the glucose-related substance. In a particular embodiment, the promotion of sugar uptake can be confirmed, as described in Examples mentioned later, by treating cells (e.g., adipocytes) with the composition B of the present invention, then administering 2DG thereto, incubating the cells for a predetermined time (e.g., approximately 5 to approximately 15 minutes, preferably approximately 10 minutes), and then detecting and quantifying 2DG6P present in the cells with a detection reagent. The rate of change in index (the rate of increase in intracellular 2DG6P level in the method using 2DG) may be, for example, 5% or more, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, or 100% or more based on an index obtained under the same conditions as above except that the composition B of the present invention is not employed. A larger value is more preferred.

The composition B of the present invention can also be employed in treatment of a condition that is improved by promotion of sugar uptake. Thus, the present invention also relates to a composition for use in the treatment of a condition that is improved by promotion of sugar uptake, the composition comprising steviol and/or a source of steviol (e.g., steviol glycoside). Examples of the condition that is improved by promotion of sugar uptake include, but are not limited to, diabetes mellitus, abnormal glucose metabolism, and hyperglycemia. In one embodiment, the condition that is improved by promotion of sugar uptake is a condition low responsive to insulin. In another embodiment, the composition B of the present invention is employed in a subject without diabetes mellitus that responds to insulin therapy (e.g., type 1 diabetes mellitus). In another embodiment, the composition B of the present invention is employed in a subject that manifests insulin resistance. The sugar uptake-promoting effect of steviol revealed this time is not mediated by insulin. Therefore, the composition B of the present invention can exert its effects in an insulin-independent manner. Thus, the composition B of the present invention may be employed even for the condition, as described above, on which insulin cannot be expected to have a therapeutic effect.

The compositions A and B of the present invention (hereinafter, may be collectively referred to as the "composition of the present invention") may be a composition of any form, and may be, for example, a solid (e.g., powdery), gel-like, or liquid composition. Also, the composition of the present invention can be in various forms. The composition of the present invention can assume, for example, a form such as a food, a food additive, a medicament, a quasi-drug, or a cosmetic, or can be employed as a starting material for producing such a product. Thus, the present invention relates to a food, a food additive, a pharmaceutical product, a quasi-pharmaceutical product or a cosmetic to be employed in use selected from suppression of fat accumulation, promotion of lipolysis, treatment of a condition involving fat accumulation, reduction in body fat, promotion of sugar uptake, reduction in blood glucose level, improvement in sugar metabolism and treatment of a condition that is improved by promotion of sugar uptake, or a starting material of the above product, the product or the starting material comprising steviol and/or a source of steviol (e.g., steviol glycoside).

In the present invention, the food is a generic name for an eatable product, and includes a food prescribed in each country or region. The food according to the present invention includes, for example, a general food (which includes a health food such as a functional food, a dietary supplement, a health supplement, and an organic food), a food with health claims (a food for specified health use, a food with functional claims, a food with nutrient function claims, etc.), and a food for special dietary use (a food for specified health use, a food for sick people, dry milk for expectant and nursing mothers, an infant formula, a food for a dysphagic patient, etc.) as defined in Japan. The food according to the present invention also includes an infant food, a formula for a baby born prematurely, a food for aged people, a nursing care food, a medical food, etc.

Nutritional supplement foods refer to foods in which a specific nutritional ingredient is fortified. Health foods refer to foods that are healthful or are considered good for health, and include nutritional supplement foods, natural foods, diet foods. Functional foods refer to foods for supplying a nutritional ingredient that fulfills regulatory functions of the body. Foods for infants refer to foods that are provided to children aged up to about six. Geriatric foods refer to foods processed to be digested and absorbed more easily than non-processed foods. Infant milk formulas refer to milk formulas to be provided to infants aged up to about one. Premature infant milk formulas refer to milk formulas to be provided to premature infants until about six months after birth.

Forms of the foods are not particularly limited, and various forms may be taken. Examples of such forms include beverages, supplements, non-beverage foods, and the like. The beverages may be either of alcoholic beverages or non-alcoholic beverages.

Examples of the alcohol-free beverage include alcohol-free beer, malt beverages, lactic acid bacteria beverages, fermented beverages, cocoa beverages, sport drinks, energy drinks, tea-based beverages (e.g., tea beverages such as green tea, oolong tea, brown rice tea, and black tea, and herbal infusion beverages such as barley tea, Job's tears tea, sesame barley tea, buckwheat tea, cherry tea, Amacha (Japanese herbal tea made from fermented leaves of Hydrangea macrophylla var. thunbergii), and herbal tea), soft drinks, coffee beverages (packed coffee, liquid coffee, etc.), carbonated beverages (e.g., cola flavor beverages, clear carbonated beverages, ginger ale, fruit juice-based carbonated beverages, milk-containing carbonated beverages and sugar-free carbonated beverages), functional beverages (e.g., sport drinks, energy drinks, health support beverages and pouched jelly beverages), fruit juice/vegetable-based beverages (e.g., 100% fruit juice beverages, fruit-containing beverages, low-fruit juice content soft drinks, fruit grain-containing fruit beverages and fruit pulp beverages), dairy beverages (e.g., milk, drink yogurt, lactic acid bacteria beverages and milk-containing soft drinks), soy milk beverages (e.g., soy milk and soybean beverages), and flavor water.

Alcoholic beverages refer to beverages that contain alcohol raw material, and may be chuhai. Examples of the alcohol raw material include fermented liquors, distilled liquors, and mixed liquors. Examples of the fermented liquor include wine and beer. Examples of the distilled liquor include spirits (such as gin, vodka, rum, tequila, new spirits (alcoholic beverages mixed with less than 10% of unblended whiskey), and alcohol for raw material), liqueurs, whiskeys (such as whiskey and brandy), and shochu. Here, alcoholic beverages may be those containing alcohol at a detectable level and contain, for example, 1% by volume or more, 2% by volume or more, 3% by volume or more, 4% by volume or more, and 5% by volume or more of alcohol.

The beverage includes beverages having various calories. The beverage is preferably a low-calorie beverage (less than 20 kcal/100 mL) or a non-caloric beverage (less than 5 kcal/100 mL).

Non-beverage foods include, for example, a noodle (such as soba noodle, udon, Chinese noodle, instant noodle); tofu; a confectionery (such as a candy, a gum, a chocolate, a snack, a biscuit, a cookie, a gummy, a cake, a wafer, a sweet bun, a chocolate, and a Japanese sweet); a bread; a marine or livestock processed food (such as a kamaboko, a ham, and a sausage); a dairy product (such as a fermented milk, a butter, a cheese, a yogurt, and a ghee); a fat and oil, and an fat-and-oil processed food (such as a salad oil, a tempura oil, a margarine, and a mayonnaise, a shortening, a whipped cream, a dressing, and a fat spread); a condiment (such as a sauce and a baste); a cooked or semi-cooked product (such as a chanpuru); a retorted food (such as a curry, a stew, a bowl, a porridge, and a rice porridge); a chilled sweet (such as an ice cream, a sorbet, and a shaved ice); a powdered food (such as a powdered beverage and a powdered soup); and an enteric fluid food.

Preferable food in the present invention includes a beverage, a supplement, and the like.

The food additive is a product that is added to a food ingredient in producing a food, and is capable of assuming, for example, a form such as a powder, granules, a liquid or a paste.

Examples of the medicament include various dosage forms. Examples of the dosage form suitable for oral administration include, but are not limited to, powders, granules, tablets, capsules, solutions, suspensions, emulsions, gels, chewables, and syrups. Examples of the dosage form suitable for parenteral administration include injections (e.g., injectable solutions, injectable suspensions, injectable emulsions, and injections of type of preparation in use), ointments, creams, lotions, wet dressings, patches, eye drops, nasal drops, inhalants, and sprays.

Examples of the quasi-drug include, but are not limited to, forms such as dentifrices, mouth fresheners, diaphoretics, cosmeceuticals, hair coloring products, and sanitary napkins.

Examples of the cosmetic include, but are not limited to, face washes, makeup removers, cosmetic lotions, beauty essences, packs, and sunscreens.

The composition of the present invention may contain a component other than steviol and a source of steviol (e.g., steviol glycoside). Such a component may differ depending on the form, use, usage, etc., of the composition of the present invention. Non-limiting examples thereof include a sweetener, an excipient, a binder, a disintegrant, a coating agent, a lubricant, a colorant, a taste and flavor modifier, a stabilizer, an absorption enhancer, a pH adjusting agent, a preservative, an anti-oxidant, a fragrance, a vitamin, a trace metal ingredient, and the like.

Examples of the sweetener include natural sweeteners, sugar alcohols, artificial sweeteners, high-intensity sweeteners, and low-calorie sweeteners. More specific examples thereof include glucose, D-fructose, galactose, mannose, ribose, xylose, maltose, sucrose, lactose, arabinose, rare sugar (e.g., D-tagatose, D-sorbose, D-allulose (D-psicose), L-fructose, L-allulose (L-psicose), L-tagatose, L-sorbose, altrose, D-allose, talose, idose, and gulose), peptide-based sweeteners (such as aspartame, neotame and advantame); sucrose derivatives (such as sucralose); synthetic sweeteners (such as acesulfame K, saccharin, advantame, sodium cyclamate, dulcin), sugar alcohols (such as erythritol, xylitol, sorbitol, maltitol, and mannitol), monellin, curculin, brazzein, thaumatin; taumarin, mabinlin, brazzein, pentadin, hernandulcin, 4β-hydroxyhernandulcin, miraculin, glycyrrhizin, phyllodulcin; Siraitia grosvenorii (luo han guo) extract, Glycyrrhiza glabra (glycyrrhiza) extract, Rubus suavissimus S. Lee (tencha) extract, Hydrangea macrophylla var. thunbergii (sweet tea) extract, Sclerochiton ilicifolius extract, Thaumataococcus daniellii Benth (Katemfe) extract, Dioscoreophyllum volkensii (serendipity berry) extract, Curculigo latifolia (Curculigo) extract, Richadella dulcifica (miracle fruit) extract, Pentadiplandra brazzeana (white liana) extract, Capparis masaikai (Mabinlang) extract, Lippia dulcis (Mexican sweet herb) extract, neohesperidin dihydrochalcone, Palatinit, high fructose liquor; fructose-glucose liquor; glucose-fructose liquor; oligosaccharide; honey; sugar cane juice (brown sugar syrup); sugar (white sugar, soft brown sugar, brown sugar, refined Japanese sugar, and the like); maple syrup; molasses; and starch syrup.

In the case of employing the composition of the present invention as a food, a food additive may be contained as the further component. Examples of the food additive include an excipient (such as wheat starch, corn starch, cellulose, lactose, sucrose, mannitol, sorbitol, xylitol, a pregelatinized starch, casein, magnesium aluminate silicate, and calcium silicate); a binder (such as pregelatinized starch, hydroxypropyl methyl cellulose, and polyvinylpyrrolidone; a disintegrant such as cellulose, hydroxypropyl methyl cellulose, and corn starch); a fluidizer (such as light anhydrous silicic acid); a fat and oil (for example, a vegetable oil such as soybean oil, sesame oil, olive oil, linseed oil, perilla oil, rapeseed oil, coconut oil, and corn oil, or an oil derived from animals or fish); a nutrient (such as various kind of minerals, various kind of vitamins, and an amino acid); a flavor; a sweetener; a taste modifier; a colorant; a solvent (such as ethanol); salts; a pH adjusting agent; a buffer; an anti-oxidant; a stabilizer, a gelling agent; a thickener; a lubricant; an encapsulant; a suspending agent; a coating agent; a preservative; and an emulsifier. The food additive may be used singly or in combination of two or more.

In the case of employing the composition of the present invention as a medicament or a quasi-drug, pharmaceutically acceptable additives (e.g., surfactants, carriers, diluents, and excipients) may be contained as other components. The pharmaceutically acceptable additives are well known in the medical field, and described in, for example, Remington's Pharmaceutical Sciences, 18th Ed., Mack Publishing Co., Easton, PA (1990), which is incorporated herein by reference in its entirety.

The composition of the present invention can be produced, for example, by mixing steviol and/or a source of steviol (e.g., steviol glycoside), if necessary, with other components as mentioned above, and processing the resultant into a desired form.

The composition of the present invention can be employed in a manner suitable for each form. For example, the food can be orally ingested as well as can be ingested through a parenteral nutritional pathway such as a transnasal or enteral pathway. The food additive can be employed in a state contained in a food, through the same pathway as in the food. The medicament can be administered through an administration route appropriate for a dosage form, for example, a route such as, but not limited to, oral, buccal, intraoral, intravenous, intramuscular, subcutaneous, intracutaneous, local, rectal, intra-articular, intracapsular, intra-arterial, intraportal, intraventricular, transmucosal, percutaneous, intranasal, intraperitoneal, intra-airway, intratumoral, intracerebral, intrathecal, intracerebroventricular, intrapulmonary and intrauterine routes. Both the quasi-drug and the cosmetic can be employed by use methods appropriate for their forms, for example, application to the skin or gargling.

The intake amount (dose or dosage) of steviol and/or the source of steviol (e.g., steviol glycoside) is not particularly limited and can be appropriately selected according to the age, body weight, health condition, etc. of a recipient subject. The daily intake may be, for example, 0.001 mg or more (e.g., 0.001 to 10,000 mg), 0.01 mg or more (e.g., 0.05 to 5,000 mg), 0.1mg or more (e.g., 0.5 to 1,000 mg) or 1 mg or more (e.g., 5 to 500 mg).

The timing of use of the compositions of the present invention is not particularly limited, and may be, for example, before, during, after, or between meals, or before bedtime.

The product comprising the composition of the present invention may indicate each type of use or function. Examples of such an indication include suppression of fat accumulation, promotion of lipolysis, treatment of a condition involving fat accumulation, reduction in body fat, promotion of sugar uptake, reduction in blood glucose level, improvement in sugar metabolism and treatment of a condition that is improved by promotion of sugar uptake, and identifications that can be regarded as being equivalent thereto. The condition involving fat accumulation and the condition that is improved by promotion of sugar uptake are as described above with respect to the composition of the present invention.

Herein, the term "subject" means any individual organism, preferably animal, more preferably mammalian, further preferably human individual to which the composition of the present invention is able to provide a desired effect. The subject may be healthy (e.g., have no particular or any disease) or may be affected by some disease. When the treatment of a disease is intended, for example, the subject typically means a subject affected by the disease or having a risk of being affected by the disease.

Herein, the term "treatment" includes every type of medically acceptable prophylactic and/or therapeutic intervention aimed at cure, transient remission or prevention, etc. of a disease. The "treatment" includes medically acceptable intervention for various purposes including, for example, the delay or arrest of progression of a disease, the regression or disappearance of a lesion, the prevention of onset of the disease or the prevention of recurrence of the disease. Thus, the composition of the present invention can be used in the treatment and/or the prevention of a disease.

Another embodiment of the present invention relates to a method of suppressing fat accumulation, promoting lipolysis, treating a condition involving fat accumulation, reducing body fat, promoting sugar uptake, reducing a blood glucose level, improving sugar metabolism, or treating a condition that is improved by promotion of sugar uptake in a subject in need thereof, comprising administering an effective amount of steviol and/or a source of steviol (e.g., steviol glycoside) to the subject.

The effective amount of steviol and/or the source of steviol (e.g., steviol glycoside) in the method of the present invention includes an amount of steviol and/or the source of steviol (e.g., steviol glycoside) capable of achieving an effect related to the method, and can be determined by the evaluation approach described above with respect to the composition of the present invention, an experiment using a known animal model related to the target disease, or condition. In a particular embodiment, the effective amount of steviol and/or the source of steviol (e.g., steviol glycoside) in the method of the present invention can be, for example, 0.001 mg or more (e.g., 0.001 to 10,000 mg), 0.01 mg or more (e.g., 0.05 to 5,000 mg), 0.1mg or more (e.g., 0.5 to 1,000 mg) or 1 mg or more (e.g., 5 to 500 mg).

Other features, for example, steviol and the source of steviol (e.g., steviol glycoside), and the target disease or condition, in the method of the present invention are as described above about the composition of the present invention.

Another embodiment of the present invention relates to steviol and/or a source of steviol (e.g., steviol glycoside) for use in the suppression of fat accumulation, in the promotion of lipolysis, in the treatment of a condition involving fat accumulation, in the reduction in body fat, in the promotion of sugar uptake, in the reduction in blood glucose level, in the improvement in sugar metabolism and in the treatment of a condition that is improved by promotion of sugar uptake. Each feature according to this embodiment is as described above with respect to the composition of the present invention.

Another embodiment of the present invention relates to use of steviol and/or a source of steviol (e.g., steviol glycoside) in the manufacture of a medicament for suppressing fat accumulation, promoting lipolysis, treating a condition involving fat accumulation, reducing body fat, promoting sugar uptake, reducing blood glucose level, improving sugar metabolism and treating a condition that is improved by promotion of sugar uptake. Each feature according to this embodiment is as described above with respect to the composition of the present invention.

### EXAMPLES

Hereinafter, Examples, etc. related to the present invention will be described. However, the present invention is not limited by these specific embodiments.

### [Example 1] Evaluation of cytotoxicity of steviol to adipocyte

Steviol was obtained by the following approach. Sodium periodate was added to a container containing stevioside (Cat. No. S0594, Tokyo Chemical Industry or Cat. No. S623, AK Scientific) and purified water, and the mixture was stirred overnight at room temperature. Potassium hydroxide was added thereto at internal temperature of 5 to 10°C under ice cooling, and the mixture was heated at internal temperature of 100 to 102°C for 2 hours. After confirmation of the completion of the reaction by HPLC, acetic acid was added thereto at internal temperature of 10 to 13°C under ice cooling. Next, extraction with ethyl acetate was performed three times, and the obtained organic layer was washed with a 10% aqueous sodium sulfite solution and subsequently with saline. The organic layer was dried over anhydrous magnesium sulfate and then concentrated under reduced pressure at 40°C to obtain a crude product of a yellow solid. Methanol was added thereto, and the mixture was stirred for approximately 15 minutes. Then, purified water was added thereto, and a solid was collected by filtration. The solid collected by filtration was dried overnight at 50°C to obtain a white solid. This solid was dissolved in acetone at 50°C, followed by clarifying filtration. The filtrate was concentrated, and the obtained solid was dried under reduced pressure overnight at 50°C to obtain steviol of interest (purity: 98% (HPLC), acetone: 0.5 wt%). Steviol thus obtained was employed in the following experiments.

5 × 10⁵ normal human subcutaneous preadipocytes (Cat. No. PT-5020, Lonza Group AG) were precultured for 6 days in a T75 flask using a growth medium (PBM-2 medium supplemented with 10% FBS, 2 mM L-glutamine, 50 µg/mL gentamycin, and 37 ng/mL amphotericin-B, Cat. No. PT-8002, Lonza Group AG) in a CO₂ incubator (37°C, 5% CO₂; the same holds true for the description below).

The cells were detached using 0.25% trypsin-EDTA, seeded at 10,000 cells/100 µL/well in a 96-well plate using the growth medium, and cultured overnight in a CO₂ incubator. On the next day, the medium was replaced with 100 µL of a differentiation medium (PBM-2 medium supplemented with 10% FBS, 2 mM L-glutamine, 50 µg/mL gentamycin, 37 ng/mL amphotericin-B, insulin, dexamethasone, indomethacin, and 3-isobutyl-1-methylxanthine (IBMX), Cat. No. PT-8002, Lonza Group AG; the same holds true for the description below) supplemented with varying concentrations of steviol (0.001, 0.01, 0.1, 1, 10 or 100 µg/mL, dissolved in 0.1% DMSO (final concentration)). The cells were cultured for 10 days in a CO₂ incubator to induce their differentiation into adipocytes. On the fourth and seventh days after the differentiation induction, the medium was replaced with a fresh medium (100 µL) having the same composition as above. A vehicle control composed of a differentiation medium supplemented with 0.1% DMSO (final concentration) was used as a negative control. Viable cell count measurement was performed by the WST-8 method. Specifically, the medium of each well after culture was replaced with a differentiation medium (100 µL) supplemented with a 1/10 amount of viable cell count measurement reagent SF, and the cells were incubated in a CO₂ incubator. 30 minutes later and 90 minutes later, absorbance (450 nm, reference wavelength: 630 nm) was measured using a plate reader (VARIOSKAN FLASH, Thermo Fisher Scientific Inc.), and absorbance difference per 60 minutes was regarded as a relative viable cell count (Control =100%).

As shown in the results of Table 1 and Figure 1, no particular cytotoxicity to be noted was found when 100 µg/mL steviol was added.

**Table 1 Results of cytotoxicity to adipocytes**

| | Control | Steviol (µg/mL) | | | | |
|---|---|---|---|---|---|---|
| | | 0.01 | 0.1 | 1 | 10 | 100 |
| Relative viable cell count (%) | 100 | 101.8 | 101.7 | 95.0 | 103.6 | 93.1 |
| Standard error | 1.3 | 2.5 | 1.9 | 3.4 | 1.7 | 1.6 |

### [Example 2] Evaluation of lipolysis-promoting effect of steviol on adipocyte

Normal human subcutaneous preadipocytes were precultured using the growth medium in the same manner as in Example 1. The cells were detached using 0.25% trypsin-EDTA, seeded at 10,000 cells/100 µL/well to a 96-well plate using the growth medium, and cultured overnight in a CO₂ incubator. On the next day, the medium was replaced with the differentiation medium (100 µL), and the cells were cultured for 10 days in a CO₂ incubator to induce their differentiation into adipocytes.

On the fourth and seventh days after the differentiation induction, the medium was replaced with a fresh differentiation medium (100 µL). Next, the medium was replaced with a differentiation medium supplemented with varying concentrations of steviol (10, 30 or 100 µg/mL, dissolved in 0.1% DMSO (final concentration)), and the cells were cultured for 24 hours in a CO₂ incubator. Then, a free glycerol level in a culture supernatant was measured using Adipolysis Assay Kit (Cat. No. 10009381, Cayman Chemical Co.) according to the manufacturer's protocol. For the measurement, absorbance (540 nm) was measured using a plate reader (VARIOSKAN FLASH, Thermo Fisher Scientific Inc.), and a relative free glycerol concentration was calculated. A differentiation medium supplemented with 10 µM isoproterenol (ISP) was used as a positive control, and a vehicle control (DMSO) composed of a differentiation medium supplemented with 0.1% DMSO (final concentration) and a no-addition control composed of only the differentiation medium were used as negative controls. Significant difference among the test groups to be compared (no-addition group vs positive control group, vehicle control group vs steviol addition group) was tested by the Student's t test. The two-tailed test was conducted, and P < 0.05 was regarded as having significant difference. As shown in the results of Table 2 and Figure 2, steviol promoted lipolysis in adipocytes in a concentration-dependent manner.

**Table 2 Promotion of lipolysis in adipocytes**

| | No addition | ISP | DMSO | Steviol (µg/mL) | | |
|---|---|---|---|---|---|---|
| | | | | 10 | 30 | 100 |
| Relative lipolysis level (%) | 100 | 184.4 | 100 | 99.5 | 115.5 | 123.4 |
| Standard error | 1.1 | 3.2 | 1.8 | 4.9 | 1.1 | 3.1 |

### [Example 3] Evaluation of fat accumulation-suppressing effect of steviol on adipocyte

Normal human subcutaneous preadipocytes were precultured using the growth medium in the same manner as in Example 1. The cells were detached using 0.25% trypsin-EDTA, seeded at 10,000 cells/100 µL/well to a 96-well plate using a growth medium, and cultured overnight in a CO₂ incubator.

On the next day, the medium was replaced with 100 µL of a differentiation medium supplemented with varying concentrations of steviol (12.5, 25, 50, 100 or 200 µg/mL, dissolved in 0.1% DMSO (final concentration)). The cells were cultured for 10 days in a CO₂ incubator to induce their differentiation into adipocytes. On the fourth and seventh days after the differentiation induction, the medium was replaced with a fresh medium (100 µL) having the same composition as above. A differentiation medium supplemented with 25 mM LiCl (Cat. No. SML0559, Sigma-Aldrich Co., LLC) was used as a positive control, and a differentiation medium supplemented with 0.1% DMSO (final concentration) was used as a negative control. Cells cultured in a growth medium were used as an untreated control (Untreated). The cells thus cultured were subjected to oil red staining. Specifically, the cells were washed twice with 100 µL of DPBS (Cat. No. 14190250, Thermo Fisher Scientific Inc.). Then, the cells were fixed (room temperature, 10 min) in 100 µL of a 10% formalin solution (Cat. No. 061-00416, FUJIFILM Wako Pure Chemical Corp.). The cells were washed twice with 100 µL of DPBS, then incubated for 1 minute in 100 µL of 60% 2-propanol (Cat. No. 166-04836, FUJIFILM Wako Pure Chemical Corp.) that replaced the medium, and stained for 15 minutes in 100 µL of an oil red staining solution (Cat. No. O0625, Sigma-Aldrich Co., LLC) that replaced the 2-propanol solution. The cells were washed once with 100 µL of 60% 2-propanol and washed twice with 100 µL of DPBS. Then, cell images were taken in 100 µL of DPBS. After subsequent replacement with 120 µL of 100% 2-propanol, oil red was extracted by incubation for 5 minutes, and 100 µL of the extracts was transferred to each well of a fresh 96-well plate. Absorbance (520 nm) was measured using a plate reader (VARIOSKAN FLASH, Thermo Fisher Scientific Inc.), and a relative fat accumulation level was calculated. Significant difference of differentiation non-induced group vs differentiation induced group (negative control group) was tested (two-tailed), and P < 0.05 was regarded as having significant difference. Also, significant difference of negative control group vs steviol addition group/positive control group was tested (two-tailed) by the Dunnett test, and P < 0.05 was regarded as having significant difference. As shown in the results of Table 3 and Figure 3, steviol suppressed fat accumulation in adipocytes in a concentration-dependent manner.

**Table 3 Suppression of fat accumulation in adipocytes**

| | Untreated | DMSO | LiCl | Steviol (µg/mL) | | | | |
|---|---|---|---|---|---|---|---|---|
| | | | | 12.5 | 25 | 50 | 100 | 200 |
| Relative fat accumulation level (%) | 52.9 | 100 | 56.3 | 98.3 | 91.3 | 80.7 | 70.8 | 40.0 |
| Standard error | 3.4 | 4.1 | 3.5 | 4.4 | 5.7 | 0.8 | 2.0 | 1.3 |

### [Example 4] Evaluation of sugar uptake-promoting effect of steviol on adipocyte

Normal human subcutaneous preadipocytes were precultured using the growth medium in the same manner as in Example 1. The cells were detached using 0.25% trypsin-EDTA, seeded at 10,000 cells/100 µL/well to a 96-well plate using the growth medium, and cultured overnight in a CO₂ incubator. On the next day, the medium was replaced with the differentiation medium (100 µL). The cells were cultured for 10 days in a CO₂ incubator to induce their differentiation into adipocytes. On the fourth and seventh days after the differentiation induction, the medium was replaced with a fresh differentiation medium (100 µL). Next, the medium was replaced with 100 µL of a serum-free medium (PBM-2 medium supplemented with 2 mM L-glutamine, 50 µg/mL gentamycin, and 37 ng/mL amphotericin-B, Cat. No. PT-8002, Lonza Group AG), and the cells were cultured for 18 hours in a CO₂ incubator. Subsequently, the medium was replaced with 100 µL of a glucose-free serum-free medium (DMEM, no glucose, Cat. No. 11966025, Thermo Fisher Scientific Inc.) supplemented with varying concentrations of steviol (10, 30 or 100 µg/mL, dissolved in 0.1% DMSO (final concentration)), and the cells were cultured for 4 hours in a CO₂ incubator. A glucose-free serum-free medium supplemented with 100 µM acadesine (AICAR, Cat. No. A9978, Sigma-Aldrich Co., LLC) was used as a positive control, and a vehicle control composed of a glucose-free serum-free medium supplemented with 0.1% DMSO (final concentration) and a no-addition control composed of only a glucose-free serum-free medium were used as negative controls. After culture, intracellular 2-deoxyglucose-6-phosphate (2DG6P, 2-deoxyglucose (2DG) phosphorylation product) level was measured by the following procedures using Glucose Uptake-Glo Assay kit (Cat. No. J1341, Promega Corp.). The medium of each well was replaced with a DPBS solution (50 µL) supplemented with 1 mM 2DG. After incubation at room temperature for 10 minutes, a stop buffer (25 µL), a neutralization buffer (25 µL), and a 2DG6P detection reagent (100 µL) were added thereto. After incubation at room temperature for 60 minutes, luminescence intensity was measured using a plate reader (VARIOSKAN FLASH, Thermo Fisher Scientific Inc.), and relative luminescence intensity was regarded as a relative intracellular 2DG6P level. Significant difference among the test groups to be compared (no-addition group vs positive control group, vehicle control group vs steviol addition group) was tested by the Student's t test. The two-tailed test was conducted, and P < 0.05 was regarded as having significant difference. As shown in the results of Table 4 and Figure 4, steviol promoted sugar uptake in adipocytes in a concentration-dependent manner.

**Table 4 Promotion of sugar uptake in adipocytes**

| | No addition | AICAR | DMSO | Steviol (µg/mL) | | |
|---|---|---|---|---|---|---|
| | | | | 10 | 30 | 100 |
| Relative luminescence intensity (%) | 100 | 194.9 | 100 | 109.6 | 142.5 | 216.2 |
| Standard error | 6.5 | 9.1 | 13.8 | 11.3 | 12.6 | 10.6 |

### [Example 5] Evaluation of cytotoxicity of steviol to hepatocyte

5 × 10⁵ HepG2 cells (Cat. No. JCRB1054, JCRB) were precultured for 5 days in a T75 flask using a growth medium (DMEM (high glucose, Cat. No. D5796, Sigma-Aldrich Co., LLC) supplemented with 10% FBS (Cat. No. 172012, Sigma-Aldrich Co., LLC) and 1% penicillin-streptomycin (Cat. No. 09367-34, Nacalai Tesque Inc.)) in a CO₂ incubator. The cells were detached using 0.25% trypsin-EDTA, seeded at 10,000 cells/100 µL/well to a 96-well plate using the growth medium, and cultured overnight in a CO₂ incubator. On the next day, the medium was replaced with 100 µL of a growth medium supplemented with varying concentrations of steviol (0.001, 0.01, 0.1, 1, 10 or 100 µg/mL, dissolved in 0.1% DMSO (final concentration)), and the cells were cultured for 24 hours in a CO₂ incubator. A vehicle control composed of a growth medium supplemented with 0.1% DMSO (final concentration) was used as a negative control (Control). Viable cell count measurement was performed by the WST-8 method. Specifically, the medium of each well after culture was replaced with 100 µL of growth medium supplemented with a 1/10 amount of viable cell count measurement reagent SF, and the cells were incubated in a CO₂ incubator. 30 minutes later and 90 minutes later, absorbance (450 nm, reference wavelength: 630 nm) was measured using a plate reader (VARIOSKAN FLASH, Thermo Fisher Scientific Inc.), and absorbance difference per 60 minutes was regarded as a relative viable cell count. As shown in the results of Table 5 and Figure 5, no particular cytotoxicity to be noted was found when 100 µg/mL steviol was added.

**Table 5 Results of cytotoxicity to hepatocyte**

| | Control | Steviol (µg/mL) | | | | |
|---|---|---|---|---|---|---|
| | | 0.01 | 0.1 | 1 | 10 | 100 |
| Relative viable cell count (%) | 100 | 98.8 | 98.4 | 95.3 | 95.6 | 87.7 |
| Standard error | 2.1 | 2.2 | 1.6 | 0.5 | 0.3 | 2.2 |

### [Example 6] Evaluation of fat/triglyceride accumulation-suppressing effect of steviol on hepatocyte

HepG2 cells were precultured using the growth medium in the same manner as in Example 5.

The cells were detached using 0.25% trypsin-EDTA, seeded at 30,000 cells/100 µL/well to a 96-well plate using the growth medium, and cultured overnight in a CO₂ incubator. On the next day, the medium was replaced with 100 µL of a test medium (DMEM (high glucose) supplemented with 0.2 mM oleic acid (Cat. No. O3008, Sigma-Aldrich), 10% FBS and 1% penicillin-streptomycin) supplemented with varying concentrations of steviol (12.5, 25, 50, 100 or 200 µg/mL, dissolved in 0.1% DMSO (final concentration)). The cells were cultured for 24 hours in a CO₂ incubator. A test medium supplemented with 2 mM metformin (Cat. No. M2009, Tokyo Chemical Industry Co., Ltd.) was used as a positive control (Metformin), and a vehicle control composed of a test medium supplemented with 0.1% DMSO (final concentration) was used as a negative control (DMSO). Cells cultured in a test medium containing no oleic acid was used as an untreated control (Untreated). The cells thus cultured were subjected to oil red staining in the same manner as in Example 3, followed by photographing and calculation of a relative fat accumulation level. An intracellular triglyceride (TG) level in cells separately cultured under the same conditions as in the cells employed in the oil red staining was quantified using Adipogenesis Assay Kit (BioVision Inc.) according to the manufacturer's protocol. Absorbance (570 nm) was measured using a plate reader (VARIOSKAN FLASH, Thermo Fisher Scientific Inc.), and a relative TG level was calculated. Significant difference of untreated group vs fat accumulation induction group (negative control group) was tested (two-tailed) by the Student's t test as to the relative fat accumulation level and the relative TG level, and P < 0.05 was regarded as having significant difference. Also, significant difference of negative control group vs steviol addition group/positive control group was tested (two-tailed) by the Dunnett test, and P < 0.05 was regarded as having significant difference. The results of the fat accumulation test are shown in Table 6 and Figure 6, and the results of the TG accumulation test are shown in Table 7 and Figure 7. Steviol suppressed fat accumulation in adipocytes in a concentration-dependent manner.

**Table 6 Suppression of fat accumulation in hepatocyte**

| | Untreated | DMSO | Metformin | Steviol (µg/mL) | | | | |
|---|---|---|---|---|---|---|---|---|
| | | | | 12.5 | 25 | 50 | 100 | 200 |
| Relative fat accumulation level (%) | 52.0 | 100 | 79.4 | 98.9 | 98.1 | 86.4 | 85.6 | 83.0 |
| Standard error | 1.7 | 4.1 | 2.9 | 2.4 | 2.7 | 2.3 | 1.9 | 3.5 |

**Table 7 Suppression of TG accumulation in hepatocyte**

| | Untreated | DMSO | Metformin | Steviol (µg/mL) | | | | |
|---|---|---|---|---|---|---|---|---|
| | | | | 12.5 | 25 | 50 | 100 | 200 |
| Relative TG level (%) | 22.7 | 100 | 76.0 | 98.8 | 90.0 | 93.7 | 89.4 | 81.4 |
| Standard error | 1.0 | 1.4 | 2.6 | 1.6 | 0.6 | 2.5 | 1.8 | 2.2 |

Those skilled in the art will understand that many various modifications can be made in the present invention without departing from the spirit of the present invention. Thus, it should be understood that the modes of the present invention described in the present specification are given merely for illustrative purposes and are not intended to limit the scope of the present invention.

## Claims

1. A composition for use in the suppression of fat accumulation and/or in the promotion of lipolysis, the composition comprising steviol and/or steviol glycoside.

2. The composition for use according to claim 1 for use in the prevention of obesity, in the improvement of obesity and/or in the reduction of body fat.

3. The composition for use according to claim 1 or 2 for a subject free of diabetes mellitus that responds to insulin therapy.

4. A composition for use in the reduction of blood glucose level, in the promotion of sugar uptake and/or in the improvement of sugar metabolism in a subject having insulin resistance, the composition comprising steviol and/or steviol glycoside.

5. A composition for use in the treatment of alcoholic liver disease or nonalcoholic fatty liver disease, the composition comprising steviol and/or steviol glycoside.

6. The composition for use according to any one of claims 1 to 5 for oral ingestion.

7. The composition for use according to any one of claims 1 to 6 which is a beverage.
